# EUROPEAN PATENT APPLICATION

(11) **EP 3 517 204 A1**
(43) Date of publication of application: **31.07.2019**
(21) Application number: 18153753.1
(22) Date of filing: 26.01.2018
(51) Int. Cl.: B01J 8/06, B01J 8/00, B01J 8/02, C07C 1/02, C07C 7/00, C07C 7/144, B01D 53/22

(54) **REACTOR AND PROCESS FOR THE HYDROGENATION OF CARBON DIOXIDE**

(71) Applicant: Nederlandse Organisatie voor toegepast- natuurwetenschappelijk onderzoek TNO, 2595 DA 's-Gravenhage (NL)
(72) Inventor: GOETHEER, Earl Lawrence Vincent, 2595 DA's-Gravenhage (NL); LINDERS, Marco Johannes Gerardus, 2595 DA's-Gravenhage (NL); GARCIA MORETZ-SOHN MONTEIRO, Juliana, 2595 DA's-Gravenhage (NL); BHARDWAJ, Rajat, 2595 DA's-Gravenhage (NL)
(74) Representative: V.O.

(57) **Abstract**

The present invention is directed to a membrane reactor for the hydrogenation of carbon dioxide, said membrane reactor comprising a reaction compartment (2) comprising a catalyst bed, a permeate compartment (4) and a membrane separating the reaction compartment and the permeate compartment, wherein said permeate compartment comprises a condensing surface.

## Description

The invention is in the field of chemical reactors and chemical processes. In particular, the invention is directed to a reactor and a process for the hydrogenation of carbon dioxide into methanol and/or dimethyl ether.

The process of hydrogenating carbon dioxide into methanol and/or dimethyl ether (also referred to as DME) can *i.a.* be used to lower CO₂ concentration in the atmosphere. In addition, the process can also be used to further valorize biogas and to provide a broader applicability of said biogas. For instance, by separating biogas into CO₂ and methane, the methane can be reformed into hydrogen (H₂) and the obtained hydrogen can be allowed to react with the CO₂ to form methanol and/or DME. This concept is not limited to biogas since in principle any CO₂-containing gas can be valorized and provided with a broader applicability by hydrogenating the CO₂ therein.

The hydrogenation of CO₂ is believed to involve the following reactions:

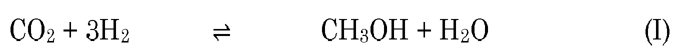

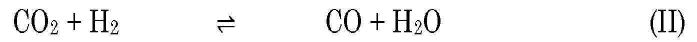

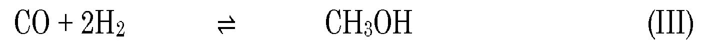

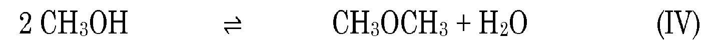

The reaction CO₂ to MeOH can proceed directly (equation I), or via the intermediate CO (equation II and III). Depending on the process parameters, the obtained MeOH can react further into DME (equation IV). As is apparent from equations I-IV, water is produced as a side product concomitantly with MeOH and/or DME. In addition, since these reactions (I-IV) are in equilibrium under the process conditions, full conversion to the products can in principle not be obtained under thermodynamic conditions. Therefore, in order to maximize the conversion into methanol and/or DME, it is preferred that at least one of the reaction products which is formed in the reaction (*i*.*e*. water, methanol and/or DME) is removed from the reaction medium.

Examples of conventional methods for the removal of the reaction products include condensation in a recycle configuration, reactive adsorption of water, and *in-situ* water-consuming reaction. US 2004/064002 discloses water vapor permeation through a membrane to remove the water from a reaction of MeOH to DME. In WO2015/030578, a reactor and a process are described comprising two stages including a zone or stage wherein a liquid condensate of methanol and water is condensed. However, all these methods are associated with several drawbacks.

For instance, a recycle configuration results in a bulky process design with installation of separate equipment, piping and control, while the use of solid system for the adsorption or reactive-consumption of water pose challenging regeneration and handling, also making the overall process bulky and expensive. Furthermore, a solid adsorption system involves regeneration which leads to a discontinuous type of operation and higher temperatures are needed for regeneration. Although the use of water vapor permeation through a membrane is elegant, this conventional method is associated with concomitant removal of H₂ from the reaction and decrease of the overall conversion. The combined condensation of methanol and water as disclosed in WO2015/0305578 requires a subsequent separation of the water and methanol in order to obtain dry methanol and results in a liquid methanol product, whereas it may be preferred to maintain a gaseous methanol product if is going to sequentially be converted into products in a gaseous phase reaction. This process is therefore relative energy consuming.

It is an object of the present invention to provide a process and reactor, in particular for the hydrogenation of carbon dioxide, that addresses at least one of these drawbacks.

A further object of the present invention is to provide an improved hydrogenation of carbon dioxide, by enhancing reaction rates towards the production of methanol and/or DME, improving catalyst lifetime, improving reactor design and intensification of process of production of methanol and/or DME from several steps to single reaction-separation-removal unit, and/or by providing a conversion beyond equilibrium constraints due to selective removal of at least one reaction product from the reaction mixture.

The present inventors found that these objects can at least partially be met by a combination of permeation of the formed water through a membrane and condensation of said water after permeation. This provides a method to selectively remove the water from the process, without the requirement of bulky process design with installation of separate equipment. The permeation and condensation are carried out in the same reactor, which reactor is another aspect of the present invention (*vide infra*). In particular, this method enables the *in-situ* removal of condensed water from the reaction within the same reactor in which the hydrogenation reaction is carried out.

Accordingly, the present invention is directed to a process for the hydrogenation of carbon dioxide, wherein said process comprises reacting carbon dioxide with hydrogen to form methanol and/or dimethyl ether, and water as a side product, and wherein said process further comprises removing said water from the process by a combination of permeation of said water through a membrane and condensation of the water.

In another aspect, the present invention is directed to a membrane reactor for the hydrogenation of carbon dioxide, said membrane reactor comprising a reaction compartment comprising a catalyst bed, a permeate compartment, and a water-permeable membrane that separates the reaction compartment and the permeate compartment. Said permeate compartment comprises a condensing surface such that the reactor is particularly suitable for carrying out the method of the present invention. The condensing surface is configured such that during operation of the reactor, the water can condense on the surface.

The water-permeable membrane is typically not exclusively permeable for water. For instance, hydrogen molecules, which are also present in the reaction compartment, are smaller than water molecules and may also (albeit undesirably) permeate the membrane. In fact, due to this permeation of hydrogen, substantial amounts of hydrogen removal from the reaction mixture have been observed in the aforementioned conventional methods. It is believed that the requirement of a sweeping gas stream to remove the vapor, also removes the permeated hydrogen. Advantageously, the presence of the condensing surface mitigates the requirement of the sweeping gas stream and the condensing surface selectively condensates water while hydrogen remains gaseous under the condensation and reaction conditions. With this method, separation of water from the reaction mixture and from H₂, may be carried out in one step, limiting the need of additional equipment for individual steps of separation and purification.

Typical reaction conditions include elevated temperatures and pressures. In particular, reacting carbon dioxide with hydrogen may be carried out at a temperature in the range of 150-400 °C, preferably in the range of 200 to 300 °C, more preferably about 250 °C and/or at a pressure in the range of 1-10 MPa, preferably in the range of 2-8 MPa, more preferably about 5 MPa.

The water-permeable membrane (herein also referred to as the membrane) can accordingly suitably function at these reaction conditions. Therefore, the water-permeable membrane is preferably a hydrophilic membrane, which preferably comprises a zeolite membrane, an amorphous membrane or a polymer membrane. Particularly suitable zeolite membranes comprise mordenite (MOR), ZSM-5, chabazite (CHA), silicalite-1 (SIL-1), Z4A, faujasite (FAU), Si/Al variant MFI, and the like. Suitable amorphous membranes may comprise Al₂O₃/SiO₂. The polymer membranes may comprise ceramic-supported polymers (CSP), as these are particularly suitable for functioning at elevated temperatures. Membranes that are used for the water removal in Fischer-Tropsch processes may also be suitably used for the present invention (see *e.g.* Rohde et al. Microporous and Mesoporous Materials 115 (2008) 123-136).

The catalyst bed may comprise any known catalyst that suitably catalyzes the hydrogenation of carbon dioxide (see *e.g.* Gallucci et al. Chemical Engineering and Processing 43 (2004) 1029-1036 and references therein, which are incorporated herein in their entirety). Particularly suitable catalysts comprise copper, zinc oxide, zirconia, palladium, cerium(IV) oxide or combinations thereof. The catalyst may be supported on suitable supports such as alumina or silica. Advantageously, the present invention can increase the lifetime of the catalyst due to an increased removal of the water. As such, less water may condense in the catalyst particles, which can increase both performance and lifetime.

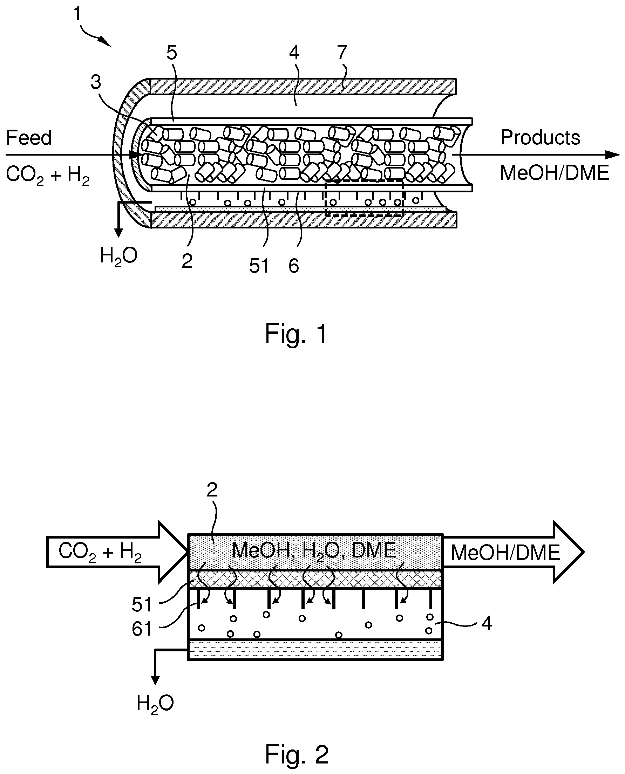

In figure 1, a particular embodiment of the reactor in accordance with the present invention is illustrated. The reactor comprises:
- an inner wall (5) bounding an inner space that defines the reaction compartment (2);
- an outer wall (7) that is arranged around said inner wall, wherein said outer wall (7) and inner wall (5) bound an outer space that defines the permeate compartment (4);
wherein said inner wall (5) comprises the water-permeable membrane (51). Preferably, as illustrated in figure 1, the inner wall and outer wall are tubular and the outer wall is co-axially arranged around said inner wall.

In a preferred embodiment, the condensing surface is a protruding surface (61), preferably a protruding surface connected to said inner wall (5) and protruding into the permeate compartment. This is illustrated in figure 2.

The protruding surface can have various shapes. For instance, the surface may be undulating or jagged to increase the total surface area. Suitable shapes may result from protruding elements such as rods, bars, blades and the like. Such protruding elements may comprise at least part of the active-cooling device such a tubing.

Preferably, the condensing surface is situated in the reactor in the vicinity of the membrane. As such, the permeated water can travel a relatively short path before it condenses. The condensing surface is generally actively cooled, meaning that it is maintained at a temperature by which water can condensate under the, generally elevated, reaction pressures. The condensing surface can be cooled actively by providing a cooling fluid stream, for instance air or (relatively) cool water, within tubing or a space near the condensing surface. It is to be understood that the cooling fluid should preferably be prevented from contacting the reactants in the reactor, such a hydrogen. The cooling fluid therefore is typically separated from the reaction compartment and the permeate compartment by a wall. For effective condensation under the reaction conditions, the condensing surface is preferably maintained at a temperature in the range of 50 to 150 °C.

The condensed water can be collected and let out the permeate compartment.

For the purpose of clarity and a concise description features are described herein as part of the same or separate embodiments, however, it will be appreciated that the scope of the invention may include embodiments having combinations of all or some of the features described.

## Claims

1. Membrane reactor (1) for the hydrogenation of carbon dioxide, said membrane reactor comprising a reaction compartment (2) comprising a catalyst bed (3), a permeate compartment (4) and a water-permeable membrane (51) separating the reaction compartment and the permeate compartment, wherein said permeate compartment comprises a condensing surface (6).

2. Membrane reactor in accordance with claim 1, which is adapted with an active-cooling device such that during operation of the reactor, water can condense on the condensing surface.

3. Membrane reactor in accordance with any of the previous claims, wherein said reactor comprises
- an inner wall (5) bounding an inner space that defines the reaction compartment (2);
- an outer wall (7) that is arranged around said inner wall,
wherein said outer wall (7) and inner wall (5) bound an outer space that defines the permeate compartment (4);
wherein said inner wall (5) comprises the water-permeable membrane (51).

4. Membrane reactor in accordance with the previous claim, wherein said inner wall and outer wall are tubular and the outer wall is at least partially co-axially arranged around said inner wall.

5. Membrane reactor in accordance with any of the previous claims, wherein the condensing surface is a protruding surface (61), preferably a protruding surface connected to said inner wall and protruding into the permeate compartment.

6. Membrane reactor in accordance with any of the previous claims, wherein said water-permeable membrane comprises a hydrophilic membrane, preferably comprising a zeolite membrane, an amorphous membrane or a polymer membrane.

7. Membrane reactor in accordance with any of the previous claims, wherein said catalyst bed comprises copper, zinc oxide, zirconia, palladium, cerium(IV) oxide or combinations thereof.

8. Process for the hydrogenation of carbon dioxide, preferably carried out in a membrane reactor in accordance with any of the previous claims, wherein said process comprises reacting carbon dioxide with hydrogen to form methanol and/or dimethyl ether, and water as a side product, and wherein said process further comprises removing said water from the process by a combination of permeation of said water through a water-permeable membrane and condensation of the water.

9. Process in accordance with the previous claim, wherein reacting carbon dioxide with hydrogen is carried out at a temperature in the range of 150-400 °C, preferably in the range of 200-300 °C, more preferably about 250 °C and/or at a pressure in the range of 1-10 MPa, preferably in the range of 2-8 MPa, more preferably about 5 MPa.

10. Process in accordance with any of claims 8-9, wherein said carbon dioxide and/or said hydrogen originate from biogas.
